# FASCICULE DE BREVET EUROPEEN

(11) **EP 4 453 540 B1**
(45) Date de publication et mention de la délivrance du brevet: **18.02.2026**
(21) Numéro de dépôt: 22844179.6
(22) Date de dépôt: 24.12.2022
(51) Int. Cl.: G01N 21/17

(54) **DISPOSITIF DE DÉTECTION PHOTOACOUSTIQUE COMPORTANT UNE MEMBRANE FORMANT UNE FACE DE CONTACT**
PHOTOAKUSTISCHE DETEKTIONSVORRICHTUNG MIT EINER MEMBRAN ZUR FORMUNG EINER KONTAKTFLÄCHE
PHOTOACOUSTIC DETECTING DEVICE COMPRISING A MEMBRANE FORMING A CONTACT FACE

(30) Priorité: 26.12.2021 FR 2114530
(43) Date de publication de la demande: 30.10.2024
(73) Titulaire: Commissariat à l'Energie Atomique et aux Energies Alternatives, 75015 Paris (FR); Eclypia, 38000 Grenoble (FR)
(72) Inventeur: JOURDE, Kévin, 38054 Grenoble Cedex 09 (FR); COUTARD, Jean-Guillaume, 38860 SAINT PANCRASSE (FR); NIORTHE, Etienne, 38054 Grenoble Cedex 09 (FR); LE ROUX MALLOUF, Thibault, 38000 GRENOBLE (FR)
(74) Mandataire: INNOV-GROUP
(86) Numéro de dépôt international: PCT/EP2022/087836
(87) Numéro de publication internationale: WO 2023/118611

(56) Documents cités:
- JP-A- 2013 244 285
- US-A1- 2007 004 974
- US-A1- 2013 298 676
- US-A1- 2014 221 810
- US-A1- 2017 156 600
- US-B2- 10 930 486

## Description

### DOMAINE TECHNIQUE

Le domaine technique de l'invention est la détection d'un analyte par détection photoacoustique.

### ART ANTERIEUR

La détection photoacoustique est basée sur la détection d'une onde acoustique générée sous l'effet de l'absorption, par un milieu analysé, d'une onde incidente électromagnétique impulsionnelle ou modulée en amplitude. L'onde acoustique est formée suite à un échauffement de molécules d'intérêt, présentes dans le milieu analysé, sous l'effet de l'absorption de l'onde incidente. L'échauffement entraîne une dilatation thermique modulée du milieu, cette dernière étant à l'origine de l'onde acoustique.

La détection photoacoustique peut être spécifique à un analyte particulier, en ajustant la longueur d'onde de l'onde incidente électromagnétique à une longueur d'onde d'absorption de l'analyte. La détection photoacoustique a été ainsi été appliquée pour détecter des espèces gazeuses dans un gaz, ou pour détecter la présence de molécules particulières dans des tissus biologiques. La longueur d'onde de l'onde incidente se situe fréquemment dans l'infra-rouge.

Un dispositif de détection photoacoustique comporte une source de lumière, par exemple une source laser, modulée en amplitude, activée selon une fréquence comprise entre plusieurs dizaines de Hz et de plusieurs dizaines de kHz. La fréquence de modulation définit la fréquence de l'onde photoacoustique résultant de l'échauffement périodique des molécules d'intérêt présentes dans le milieu à analyser. Un dispositif de détection photoacoustique comporte un détecteur acoustique configuré pour détecter l'onde photoacoustique périodique. Une fonction de réponse du dispositif photoacoustique peut être calibrée, de façon à établir une corrélation entre l'amplitude d'oscillations de pression mesurée et la quantité d'analyte dans le milieu analysé.

Un exemple de dispositif à détection photoacoustique est décrit dans US2017/156600A1, qui comporte une interface utilisée pour la transmission et le couplage d'une onde acoustique avec des détecteurs élémentaires.

Des dispositifs ont été décrits, qui permettent d'appliquer la détection photoacoustique à des milieux biologiques, par exemple pour quantifier certaines biomolécules comme le glucose. Des dispositifs, le milieu analysé s'échauffe périodiquement sous l'effet d'une illumination périodique. L'échauffement périodique du milieu se propage jusqu'à une interface entre le milieu et une cavité remplie d'air. La variation de température au niveau de l'interface se traduit par une génération d'une onde de pression périodique dans la cavité.

Les inventeurs ont conçu un dispositif de détection photoacoustique, basé sur le même principe que les dispositifs cités dans le paragraphe précédent, adapté à être appliqué sur un milieu liquide.

### EXPOSE DE L'INVENTION

Un premier objet de l'invention est un dispositif de détection photoacoustique, destiné à être appliqué, par une face de contact, contre un milieu à analyser, le dispositif comportant :
- une cavité creuse, remplie d'un gaz, ouverte sur la face de contact;
- une source de lumière, configurée pour émettre, lorsqu'elle est activée, un faisceau lumineux incident dans une bande spectrale d'émission, à travers la cavité, jusqu'à la face de contact, le faisceau lumineux incident étant impulsionnel ou modulé en amplitude ;
- un détecteur acoustique relié à la cavité, et configuré pour détecter une onde acoustique se propageant à travers la cavité ;

de telle sorte que, sous l'effet d'une illumination du milieu par le faisceau lumineux incident, le détecteur acoustique détecte une onde acoustique produite par un échauffement du milieu;
le dispositif comportant une membrane d'interface, formant la face de contact, la membrane d'interface étant configurée pour :
   - former une interface entre le gaz, remplissant la cavité, et le milieu à analyser ;
   - bloquer un passage du milieu à analyser dans la cavité, caractérisé en ce que le détecteur acoustique est configuré pour détecter une onde de pression acoustique, générée à l'intérieur de la cavité, sous l'effet d'une variation de la température de la membrane d'interface, la variation de température de la membrane d'interface étant induite par l'échauffement du milieu résultant de l'illumination du milieu.

Selon un mode de réalisation, la membrane d'interface est pleine.

Selon un mode de réalisation, la membrane d'interface comporte des ouvertures traversantes de rayon inférieur à 50 µm ou à 30 µm. La membrane peut comporter, au niveau des ouvertures traversantes, un revêtement hydrophobe.

Selon un mode de réalisation,
- la source de lumière est agencée de telle sorte que, lorsqu'elle est activée, le faisceau lumineux incident traverse la membrane d'interface avant d'atteindre le milieu à analyser;
- la membrane d'interface comporte une portion d'intersection correspondant à une partie de la membrane traversée par le faisceau lumineux ;
- au moins dans la portion d'intersection, la membrane d'interface est constituée d'un matériau de transmission présentant une transmittance, dans la bande spectrale d'émission, supérieure à 0,4.

La membrane d'interface permet ensuite d'optimiser la quantité de lumière se propageant dans le milieu. Le matériau de transmission peut être constitué d'au moins un matériau choisi parmi : Si, Ge, AIN, ZnSe, BaF₂, CaF₂, KBr, ZnS, Saphir. La membrane peut être monolithique est formée du matériau de transmission.

Selon un mode de réalisation, la membrane d'interface est amovible.

Avantageusement,
- la membrane d'interface s'étend entre une surface interne, au contact du gaz remplissant la cavité, et une surface externe, destinée à être appliquée contre le milieu à analyser ;
- la surface interne de la membrane d'interface comporte un revêtement anti-reflet ou une micro-structuration configurée pour minimiser une réflexion du faisceau lumineux;
- et/ou la surface externe de la membrane d'interface comporte un revêtement anti-reflet ou une micro-structuration configurée pour minimiser une réflexion du faisceau lumineux.

L'épaisseur de la membrane d'interface peut être comprise entre 20 µm et 1mm.

Selon un mode de réalisation,
- la cavité est délimitée par une membrane distale, la membrane distale s'étendant face à la membrane d'interface, de façon que la cavité s'étende entre la membrane distale et la membrane d'interface ;
- la source de lumière est agencée de façon de telle sorte que, lorsqu'elle est activée, le faisceau lumineux incident traverse la membrane distale avant d'atteindre la membrane d'interface.

La membrane distale peut comporter ou être constituée d'un matériau présentant une transmittance, dans la bande spectrale d'émission, supérieure à 0,4. Il peut notamment s'agir d'un matériau choisi parmi Si, Ge, AIN, ZnSe, BaF₂, CaF₂, KBr, ZnS, Saphir.

La membrane distale et la membrane de transmission peuvent être formées d'un même matériau.

Selon un mode de réalisation,
- la cavité est délimitée par une paroi distale et une paroi latérale, la paroi latérale s'étendant entre la paroi distale et la membrane d'interface ;
- la membrane s'étend entre des bords opposés de la paroi latérale.

Le volume de la cavité peut être inférieur à 50 µL.

Un deuxième objet de l'invention est un procédé de détection d'un analyte dans un milieu, l'analyte absorbant la lumière dans une longueur d'onde d'absorption, le procédé comportant :
- application d'un dispositif selon le premier objet de l'invention contre le milieu, de façon que la membrane d'interface soit au contact du milieu ;
- activation de la source de lumière, la bande spectrale d'émission contenant la longueur d'onde d'absorption de l'analyte ;
- détection d'une onde de pression photoacoustique par le détecteur acoustique et estimation d'une quantité d'analyte en fonction de l'onde de pression photoacoustique détectée, et plus précisément en fonction d'une amplitude de l'onde de pression photoacoustique.

Le milieu peut être ou comporter un liquide ou est un gel.

La source de lumière peut être impulsionnelle ou modulée en amplitude, selon une fréquence d'impulsion ou une fréquence de modulation inférieure à 500 Hz.

L'invention sera mieux comprise à la lecture de l'exposé des exemples de réalisation présentés, dans la suite de la description, en lien avec les figures listées ci-dessous.

### FIGURES

La figure 1 montre un exemple de dispositif de détection photoacoustique.
La figure 2 est un exemple de membrane d'interface.
Les figures 3A et 3B montrent un exemple d'application du dispositif contre un conduit dans lequel circule un liquide.
La figure 4 montre un autre exemple de membrane d'interface.
La figure 5 montre une modélisation de l'amplitude d'oscillations thermiques engendrées dans le milieu et la cavité lors de d'une illumination périodique du milieu (axe des ordonnées), selon un axe transversal, perpendiculaire à la membrane d'interface. L'axe des abscisses correspond à la position le long de l'axe transversal.
La figure 6 représente une modélisation d'une amplitude d'oscillations thermiques (axe des ordonnées) engendrées dans le milieu analysé et dans la cavité, sous l'effet d'une illumination périodique du milieu, en fonction de la fréquence de modulation du faisceau d'illumination (axe des abscisses).
Les figures 7A à 7C schématisent des étapes d'un procédé de fabrication de la cavité et de la membrane d'interface du dispositif.

### EXPOSE DE MODES DE REALISATION PARTICULIERS

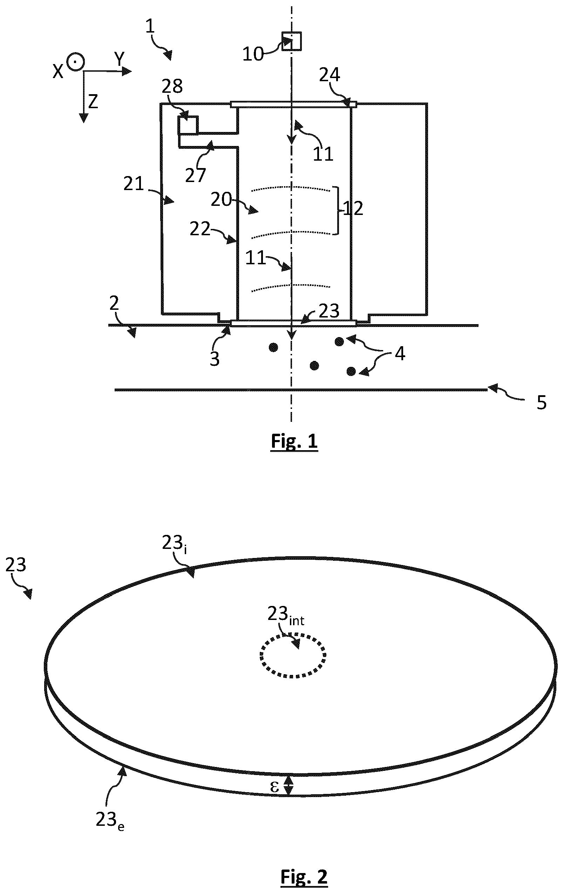

On a schématisé, sur la figure 1, un dispositif 1 permettant une mise en œuvre de l'invention. Le dispositif 1 est configuré pour être appliqué contre un milieu 2 à analyser. Le dispositif comporte une face de contact 3, destinée à être appliquée contre le milieu à analyser. Dans l'exemple représenté, le milieu 2 est un liquide s'écoulant dans un conduit 5. La circulation du liquide n'est pas nécessaire. L'invention peut s'appliquer à l'analyse d'un liquide statique disposé dans un récipient. L'invention peut également s'appliquer à l'analyse d'un solide ou d'un gel.

Le dispositif comporte une source de lumière 10, configurée pour émettre un faisceau lumineux 11 se propageant jusqu'au milieu 2 à analyser. La source de lumière 10 est impulsionnelle ou modulée en amplitude. Le faisceau lumineux 11 est émis dans une bande spectrale d'émission Δλ comportant une longueur d'onde d'absorption λₐ d'un analyte 4 présent dans le milieu. Un objectif du dispositif 1 est de détecter la présence de l'analyte 4 et éventuellement d'en estimer une concentration.

L'analyte 4 peut être une molécule présente dans un fluide, qu'il s'agisse d'un fluide corporel ou d'un fluide utilisé dans un processus industriel. Il peut par exemple s'agir de glucose, ou d'alcool, par exemple l'éthanol.

La bande spectrale d'émission Δλ s'étend de préférence dans le visible ou dans l'infra-rouge, par exemple entre des longueurs d'onde de 2 µm et de 15 µm. De préférence, la bande spectrale d'émission Δλ est suffisamment étroite, de façon que le dispositif 1 soit spécifique à un seul analyte. Lorsque l'analyte est le glucose, la bande spectrale d'émission est centrée sur une longueur d'onde d'absorption du glucose, par exemple correspondant à un nombre d'onde de 1034 cm⁻¹. La source de lumière 10 peut notamment être une source laser impulsionnelle, par exemple un laser accordable en longueur d'onde de type QCL (Quantum Cascade Laser). Selon d'autres modes de réalisation, la source de lumière peut être une source de type filament, ou une diode électroluminescente. Selon ces modes de réalisation, il est préférable d'associer la source de lumière à un filtre passe-bande pour définir une bande spectrale d'émission suffisamment étroite, et centrée sur la longueur d'onde d'absorption considérée.

Le dispositif 1 est destiné à être appliqué contre le milieu à analyser 2. Il comporte un corps 21 dans lequel une cavité a été ménagée. La cavité 20 est remplie d'un gaz, par exemple de l'air. La cavité 20 débouche sur une membrane d'interface 23. La membrane d'interface 23 forme une interface entre la cavité 20 et le milieu à analyser 2. La membrane est configurée pour bloquer un passage de liquide ou de gel entre le milieu analysé 2 et la cavité 20. Ainsi, la membrane est non poreuse à l'égard d'un liquide ou d'un gel. La membrane peut être poreuse à l'égard d'un gaz, comme décrit par la suite. Dans l'exemple représenté, la membrane affleure la surface interne du conduit dans lequel le liquide circule. La membrane peut être souple ou rigide.

La source de lumière 10 est configurée pour émettre le faisceau lumineux 11 de façon que ce dernier parvienne jusqu'au milieu à analyser 2 selon une incidence normale, ou sensiblement normale. Par sensiblement normale, on entend normale en considérant une tolérance angulaire de ± 30 °. De préférence, le faisceau lumineux 11 traverse la membrane d'interface 23 avant d'atteindre le milieu à analyser 2.

Sous l'effet de la présence d'un analyte 4 dans le milieu 2, une onde acoustique, dite onde photoacoustique 12, est formée. L'onde photoacoustique 12 est une onde acoustique formée à partir d'un échauffement périodique du milieu par le faisceau lumineux 11, ce dernier étant impulsionnel ou modulé en amplitude. L'échauffement périodique se transmet, par conduction thermique, à l'interface entre le milieu 2 et la cavité 20. Au niveau de l'interface, une onde photoacoustique périodique se forme et se propage à travers la cavité 20. L'onde photoacoustique, et notamment son amplitude, est détectée par un détecteur acoustique 28. Le détecteur acoustique 28 est relié à la cavité 20 par un canal acoustique 27. Le détecteur acoustique peut être un microphone, présentant une gamme spectrale de détection comprenant la fréquence de l'onde photoacoustique. L'onde photoacoustique est modulée en amplitude selon la fréquence d'impulsion ou de modulation d'amplitude du faisceau lumineux 11. Ainsi, au niveau du détecteur acoustique, la pression est modulée en amplitude. L'amplitude de l'onde acoustique mesurée est corrélée à la concentration de l'analyte dans le milieu.

La cavité 20 est délimitée par la membrane d'interface 23. Elle est également ménagée, dans le corps 21, entre une paroi latérale 22 et une paroi distale 24. La paroi latérale 22 s'étend autour d'un axe parallèle à un axe transversal Z, entre la membrane d'interface 23 et la paroi distale 24. L'axe transversal Z est perpendiculaire à la membrane d'interface 23. La paroi distale 24 s'étend face à la membrane d'interface 23. Dans l'exemple représenté, la paroi distale 24 est formée par une membrane, dite membrane distale 24, parallèle à la membrane d'interface 23. La source de lumière 10 est disposée en dehors de l'espace interne délimité par la cavité. Le faisceau lumineux se propage à travers la membrane distale 24, formant la paroi distale de la cavité, puis à travers la membrane d'interface 23, jusqu'au milieu analysé. Le faisceau lumineux 11 se propage de préférence à distance de la paroi latérale 22 délimitant la cavité 20, de façon à éviter un échauffement de cette dernière, ce qui conduirait à une formation d'un signal acoustique parasite, non spécifique de l'analyte recherché. C'est également la raison pour laquelle la paroi distale 24 de la cavité est préférentiellement formée par une fine membrane distale, réalisée dans un matériau considéré comme transparent dans la bande spectrale d'émission. La membrane distale présente une transmittance élevée dans la bande spectrale d'émission, de façon à maximiser la quantité de lumière se propageant vers le milieu analysé. Plus la quantité de lumière atteignant le milieu est importante, plus le dispositif est sensible.

Le volume de la cavité peut être de quelques µL ou dizaines de µL (microlitres). Afin d'augmenter l'amplitude de l'onde de pression détectée par le détecteur acoustique, on estime que le volume de la cavité est préférentiellement inférieur à 50 µL.

Le faisceau lumineux 11 atteignant le milieu 2 est progressivement absorbé dans ce dernier, notamment par l'analyte 4, selon la loi d'absorption de Beer Lambert. L'absorption est d'autant plus rapide que la concentration d'analyte absorbant le faisceau lumineux est importante. Ainsi, plus la concentration d'analyte est importante, plus l'épaisseur parcourue par le faisceau lumineux, dans le milieu est faible. L'énergie optique, absorbée par l'analyte, est restituée dans le milieu sous forme de chaleur. La chaleur diffuse dans le milieu jusqu'à la membrane d'interface. La membrane d'interface 23 subit alors une variation périodique de température, induite par l'échauffement périodique du milieu, l'échauffement périodique du milieu résultant de l'illumination périodique par le faisceau lumineux 11.

La variation périodique de température de la membrane d'interface 23 génère, à l'intérieur de la cavité, une onde de pression périodique 12, cette dernière étant détectée par le détecteur acoustique 25. C'est sous l'effet de l'échauffement périodique de la membrane 23 que l'onde de pression est formée. L'effet photoacoustique est indirect, puisqu'il se forme au niveau de la membrane d'interface, sous l'effet de la conduction de la chaleur à travers le milieu. La période de l'onde de pression formée dans la cavité correspond à la période du faisceau lumineux incident 11.

Afin que la transduction de la variation de température du milieu en variation d'amplitude de l'onde de pression soit optimale, il est préférable que la conduction thermique du matériau formant la membrane d'interface soit élevée, par exemple supérieure à 0.5 W.m⁻¹.K⁻¹ ou à 1 W.m⁻¹.K⁻¹ ou à 5 W.m⁻¹.K⁻¹ ou 10 W.m⁻¹.K⁻¹. Il est également préférable que le matériau formant la membrane ait une masse volumique faible. Il est préférable d'éviter des matériaux tels que des plastiques ou des textiles. La chaleur massique du matériau formant la membrane d'interface est de préférence faible. Enfin, la membrane d'interface est de préférence fine, comme décrit en lien avec la figure 2.

Une autre fonction de la membrane d'interface est d'éviter qu'une partie du milieu analysé ne pénètre dans la cavité. Ainsi, la membrane d'interface forme une barrière fluidique, en particulier vis-à-vis de liquides ou de gels susceptibles d'être présents dans le milieu analysé. Cela permet d'éviter une contamination de l'intérieur de la cavité. Cela permet également de maîtriser le volume de gaz à l'intérieur de la cavité. En effet, une variation du volume interne de la cavité peut entraîner un biais sur l'interprétation de la mesure résultant du détecteur acoustique. Le biais peut affecter la relation entre l'amplitude de l'onde photoacoustique et la concentration de l'analyte. Ainsi, en bloquant le passage de liquide ou de gel à l'intérieur de la cavité, la membrane d'interface permet de stabiliser la fonction de réponse du dispositif.

La figure 2 schématise un exemple de la membrane d'interface 23. La membrane d'interface 23 s'étend entre une surface externe 23ₑ, destinée à être au contact avec le milieu analysé, et une surface interne 23ᵢ, au contact du gaz présent dans la cavité. Entre la surface externe et la surface externe, la membrane s'étend selon une épaisseur ε faible, de préférence inférieure à 1 mm. Avantageusement, l'épaisseur ε de la membrane d'interface 23 est comprise entre 25 µm et 100 µm. Plus la membrane est épaisse, plus la conduction thermique jusqu'à la cavité est plus faible.

La membrane d'interface comporte une portion d'intersection 23ᵢₙₜ, correspondant à la partie de la membrane d'interface traversée par le faisceau lumineux 11. Au moins dans la portion d'intersection 23ᵢₙₜ, la membrane d'interface est formée d'un matériau présentant une transmittance élevée dans la bande spectrale Δλ du faisceau d'émission 11. Par transmittance élevée, on entend une transmittance de préférence supérieure à 0,4 voire et de préférence supérieure à 0,8, par exemple de l'ordre de 0,9 ou davantage. Par transmittance, on entend une fraction de l'intensité lumineuse transmise par la membrane d'interface 23. La membrane d'interface peut être partiellement ou entièrement formée de Si, ou autre matériau transparent à l'infra-rouge, par exemple Si poreux, Ge, AIN, ZnSe, BaF₂, CaF₂, KBr, ZnS, Saphir. Il en est de même de la membrane distale 24 précédemment décrite. Une transmittance élevée permet de maximiser la quantité de lumière atteignant le milieu.

La transmittance peut être augmentée, pour atteindre des valeurs voisines de 1, en appliquant un revêtement anti-reflet, en particulier sur la surface interne 23ᵢ et de préférence sur la surface interne 23ᵢ et sur la surface externe 23ₑ. Le revêtement antireflet peut prendre la forme d'une couche pleine « quart d'onde », déposée sous la forme de couche mince, au moins au niveau de la portion d'intersection 23ᵢₙₜ. De façon alternative, le traitement anti-reflet peut être une microstructuration de la surface interne 23ᵢ, et éventuellement de la face externe 23ₑ, au moins au niveau de la portion d'intersection 23ᵢₙₜ à travers laquelle le faisceau lumineux se propage. La microstructuration peut par exemple de former un réseau de diffraction, permettant d'optimiser la transmission de la lumière dans la bande spectrale d'émission. Un exemple de microstruration est décrit dans Douglas S. Hobbs, Brue D. MacLeod and Juanita R. Riccobono « Update on the development of high performance anti-reflecting surface relief microstructures », Proc. SPIE 6545.

La membrane d'interface peut être monolithique, en étant formée d'un seul matériau, ou composite. Lorsque la membrane d'interface est composite, elle peut comporter un matériau considéré comme suffisamment transparent dans la bande spectrale d'émission, au niveau de la portion d'intersection 23ᵢₙₜ, et un autre matériau en dehors de la portion d'intersection, par exemple un très bon conducteur thermique, de type métal tel le cuivre ou l'aluminium.

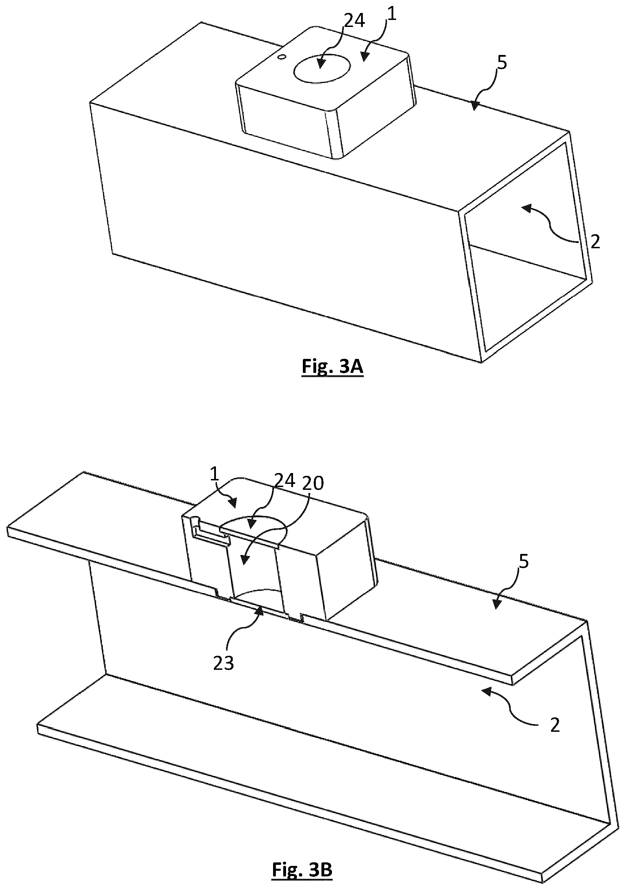

La figure 3A schématise une possibilité d'application d'un dispositif 1 tel que précédemment décrit sur un conduit 5 dans lequel circule un liquide 2. La membrane d'interface 23 est disposée dans une ouverture pratiquée dans le conduit, à l'interface entre le liquide et la cavité. La figure 3B est une vue écorchée de la figure 3A, dans un plan médian.

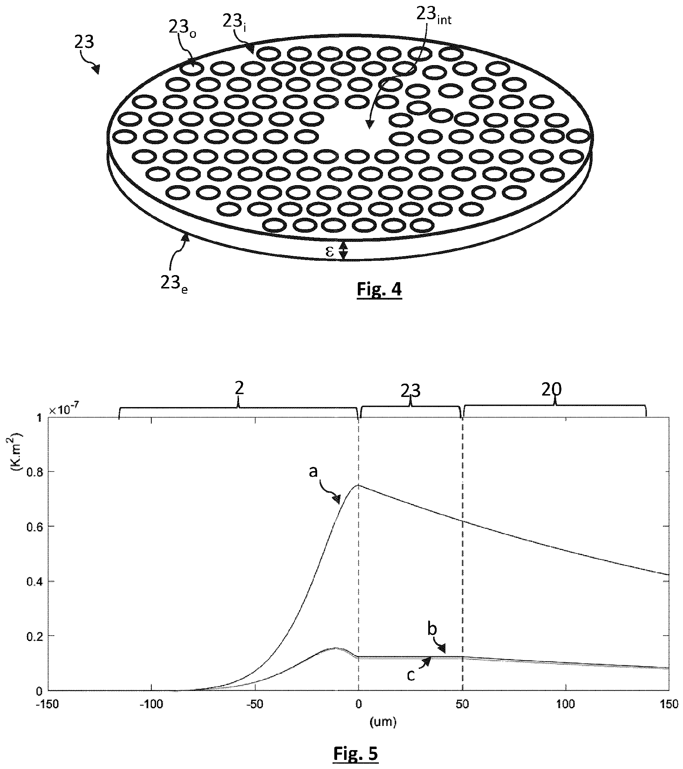

Dans l'exemple représenté sur la figure 2, la membrane d'interface 23 est pleine. Selon une possibilité, représentée sur la figure 4, la membrane d'interface 23 comporte des ouvertures traversantes 23ₒ, s'étendant à travers toute l'épaisseur de la membrane. Le rayon des ouvertures traversantes est de préférence compris entre 5 µm et 50 µm, et de préférence entre 20 et 30 µm. Les ouvertures sont avantageusement fonctionnalisées, par exemple avec un traitement hydrophobe, pour éviter un passage de liquide à travers la membrane. Une telle configuration permet de tirer profit d'une onde photoacoustique générée, dans chaque ouverture, au niveau de l'interface entre le milieu et l'air de la cavité. Selon cette possibilité, la portion d'intersection 23ᵢₙₜ de la membrane 23 est de préférence pleine, de façon que les ouvertures traversantes 23ₒ ne perturbent pas la propagation du faisceau lumineux à travers la membrane d'interface.

Dans la configuration de la figure 4, il est avantageux, mais non nécessaire, que la membrane soit un bon conducteur thermique. Ainsi, dans ce mode de réalisation, la membrane peut être un polymère comportant une multitudes d'ouvertures.

On a simulé l'amplitude d'oscillations thermiques en fonction d'une position, selon l'axe transversal Z, dans le milieu 2 et dans la cavité 20, sous l'effet d'une illumination périodique. La figure 5 représente l'amplitude des oscillations thermiques (axe des ordonnées - unité K.m²), en fonction de la position z selon un axe parallèle à l'axe Z, centré par rapport à la cavité et à la membrane. Cet axe est schématisé par des tirets mixtes sur la figure 1. Les positions z < 0 correspondent au milieu analysé 2. Les positions z > 0 correspondent au dispositif. La coordonnée z = 0 correspond à l'interface milieu/dispositif. On a tout d'abord pris en compte un dispositif sans membrane, ce qui correspond à la courbe a). On a ensuite pris en compte une membrane 23, d'épaisseur 50 µm. Les paramètres de modélisation étaient les suivants :
- échantillon : eau à une température de 25°C, épaisseur supérieure à 1 mm ;
- faisceau d'illumination : source laser de puissance 10 mW modulée à une fréquence de 100 Hz - nombre d'onde : 1035 cm⁻¹.
- constitution de la membrane : Si (courbe b) ou Ge (courbe c), sans traitement anti-reflet.

Sur la figure 5, les courbes correspondant à la membrane de Si ou de Ge sont presque confondues.

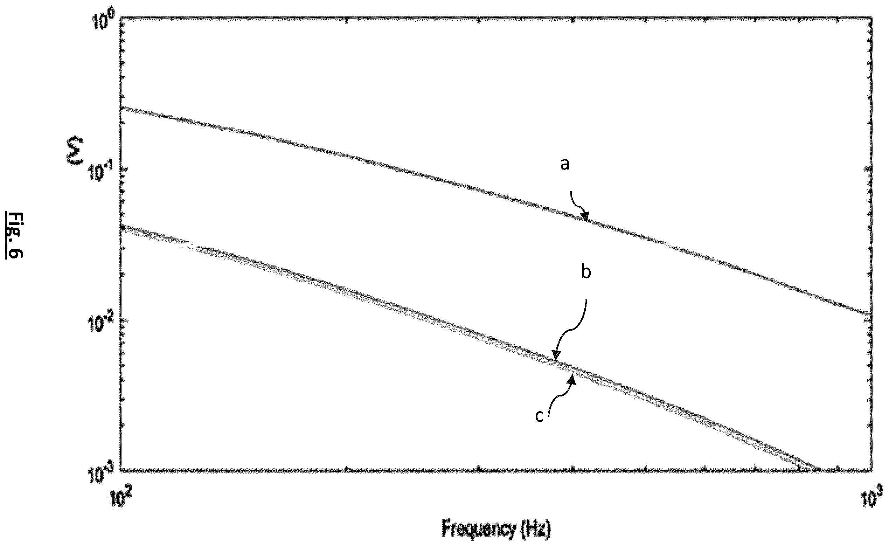

La figure 6 représente l'évolution, en fonction de la fréquence de modulation (axe des abscisses - Hz) de l'amplitude du signal, mesuré par un capteur acoustique (axe des ordonnées - unité V), représentatif de l'onde photoacoustique.

La figure 5 montre que l'effet photothermique, c'est-à-dire l'échauffement du milieu analysé par le faisceau laser, se produit sur une profondeur inférieure à 100 µm par rapport à la membrane d'interface. Par ailleurs, on observe une stabilité de la température dans l'épaisseur de la membrane. Cela est dû à la grande longueur de diffusion thermique de Si et Ge. Cela confirme que ces matériaux sont bien appropriés pour former la membrane d'interface. Par ailleurs, les valeurs très proches obtenues pour Si et Ge sont dues aux bonnes propriétés de transmission optique, dans l'infra-rouge, de ces deux matériaux.

La figure 6 montre qu'à une fréquence de modulation de 100 Hz, l'amplitude de l'onde photoacoustique est atténuée d'un facteur approximativement égal à 10 en présence de la membrane (courbes b et c) de la figure 6, par rapport à la configuration sans membrane (courbe a de la figure 6). L'atténuation induite par la membrane d'interface est attribuée à des effets de réflexions du faisceau lumineux 11 lorsqu'il se propage à travers la membrane d'interface, en particulier au niveau de la surface interne 23ᵢ et de la surface externe 23ₑ. Ces réflexions indésirables peuvent être évitées en appliquant un traitement anti-reflet sur ces deux surfaces.

Les inventeurs estiment que le recours à un traitement anti-reflet sur la surface interne et sur la surface externe est susceptible d'augmenter l'amplitude de l'onde de pression photoacoustique d'un facteur approximativement égal à 4.

On note que l'amplitude de l'onde photoacoustique diminue lorsque la fréquence de modulation augmente, et cela pour les trois configurations modélisées : sans membrane, membrane de Si et membrane de Ge. Cela est dû fait que la longueur de diffusion thermique dans la membrane, et dans l'air, diminue lorsque la fréquence augmente. Sur la figure 6, on observe que l'écart entre la courbe a (sans membrane) et les courbes b) et c) (membrane de Si ou Ge) augmente lorsque la fréquence de modulation augmente. Il est donc préférable que la fréquence de modulation soit relativement faible, par exemple inférieure à 100 Hz ou de quelques centaines de Hz. La membrane agit comme un filtre passe-bas pour l'onde photoacoustique.

La membrane d'interface 23 peut être fabriquée indépendamment de la cavité, et rapportée sur cette dernière. Elle peut être amovible.

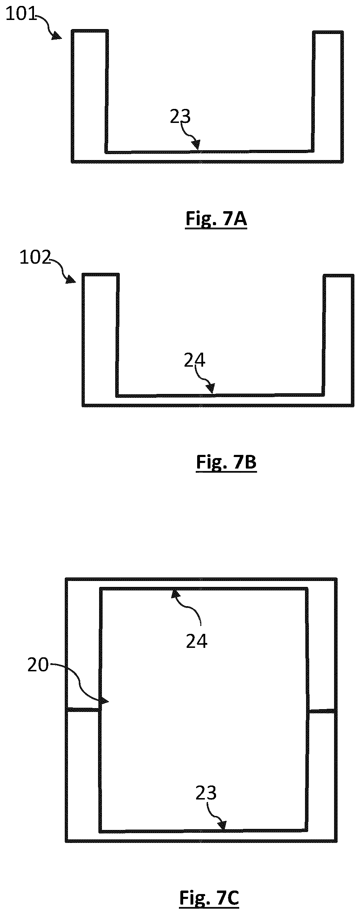

Selon une possibilité, la cavité est obtenue par un assemblage de deux substrats. Un premier substrat 101 fait l'objet d'une gravure, de façon à former une partie de la cavité, ainsi que la membrane d'interface 23: cf. figure 7A. Au niveau de la membrane d'interface 23, le substrat est aminci de façon à obtenir une membrane d'épaisseur souhaitée. Un deuxième substrat 102 fait l'objet d'une gravure, de façon à former une partie complémentaire de la cavité, et éventuellement la membrane distale 24. Cf. figure 7B. Les substrats 101 et 102 sont scellés l'un sur l'autre, de façon à former la cavité délimitée d'une part par la membrane d'interface 23 et d'autre part par la membrane distale 24 : cf. figure 7C.

Le dispositif peut comporter un matériau biocompatible, configuré pour s'étendre à l'interface entre le milieu analysé et la membrane. Le matériau biocompatible est choisi pour que la biocompatibilité soit assurée durablement, et pour qu'il permette une transmission optimale de la chaleur dégagée par le milieu analysé vers la membrane. Le matériau biocompatible peut être un métal, par exemple aluminium ou cuivre. Son épaisseur peut alors être inférieure ou de l'ordre de 1 µm. Le matériau biocompatible peut être un plastique, auquel cas l'épaisseur est de quelques dizaines ou centaines de microns.

L'invention pourra être mise en œuvre pour mesurer des concentrations de molécules d'intérêt dans un milieu, notamment un milieu liquide ou un gel. Les applications peuvent concerner le domaine de la santé, mais également des domaines industriels comme l'agroalimentaire, la pharmacie ou l'industrie chimique.

## Revendications

1. Dispositif de détection photoacoustique (1), destiné à être appliqué, par une face de contact (3), contre un milieu à analyser (2), le dispositif comportant :
- une cavité creuse (20), remplie d'un gaz, ouverte sur la face de contact;
- une source de lumière (10), configurée pour émettre, lorsqu'elle est activée, un faisceau lumineux incident (11), dans une bande spectrale d'émission (Δλ), à travers la cavité (20), jusqu'à la face de contact, le faisceau lumineux incident étant impulsionnel ou modulé en amplitude ;
- un détecteur acoustique (28) relié à la cavité;
de telle sorte que, sous l'effet d'une illumination du milieu par le faisceau lumineux incident, le détecteur acoustique détecte une onde acoustique produite par un échauffement du milieu (2);
le dispositif comportant une membrane d'interface (23), formant la face de contact, la membrane d'interface étant configurée pour :
- former une interface entre le gaz, remplissant la cavité, et le milieu à analyser ;
- bloquer un passage du milieu à analyser dans la cavité ;
**caractérisé en ce que** le détecteur acoustique est configuré pour détecter une onde de pression acoustique, générée à l'intérieur de la cavité, sous l'effet d'une variation de la température de la membrane d'interface, la variation de température de la membrane d'interface étant induite par l'échauffement du milieu résultant de l'illumination du milieu.

2. Dispositif selon la revendication 1, dans lequel la membrane d'interface (23) est pleine.

3. Dispositif selon la revendication 1, dans lequel la membrane d'interface comporte des ouvertures traversantes (23ₒ), de rayon inférieur à 50 µm ou à 30 µm.

4. Dispositif selon la revendication 3, dans lequel la membrane d'interface comporte, au niveau des ouvertures traversantes, un revêtement hydrophobe.

5. Dispositif selon l'une quelconque des revendications précédentes, dans lequel
- la source de lumière est agencée de telle sorte que, lorsqu'elle est activée, le faisceau lumineux incident traverse la membrane d'interface avant d'atteindre le milieu à analyser;
- la membrane d'interface comporte une portion d'intersection (23ᵢₙₜ), correspondant à une partie de la membrane traversée par le faisceau lumineux ;
- au moins dans la portion d'intersection, la membrane d'interface est constituée d'un matériau de transmission présentant une transmittance, dans la bande spectrale d'émission, supérieure à 0,4.

6. Dispositif selon la revendication 5, dans lequel le matériau de transmission est constitué d'au moins un matériau choisi parmi : Si, Ge, AIN, ZnSe, BaF₂, CaF₂, KBr, ZnS, Saphir.

7. Dispositif selon l'une quelconque des revendications précédentes, dans lequel la membrane d'interface est amovible.

8. Dispositif selon l'une quelconque des revendications précédentes, dans lequel
- la membrane d'interface s'étend entre une surface interne (23ᵢ), au contact du gaz remplissant la cavité, et une surface externe (23ₑ), destinée à être appliquée contre le milieu à analyser ;
- la surface interne de la membrane d'interface comporte un revêtement anti-reflet ou une micro-structuration configurée pour minimiser une réflexion du faisceau lumineux;
- et/ou la surface externe de la membrane d'interface comporte un revêtement anti-reflet ou une micro-stucturation configurée pour minimiser une réflexion du faisceau lumineux.

9. Dispositif selon l'une quelconque des revendications précédentes, dans lequel l'épaisseur de la membrane d'interface est comprise entre 20 µm et 1mm.

10. Dispositif selon l'une quelconque des revendications précédentes, dans lequel :
- la cavité (20) est délimitée par une membrane distale, la membrane distale (24) s'étendant face à la membrane d'interface, de façon que la cavité s'étende entre la membrane distale et la membrane d'interface ;
- la source de lumière (10) est agencée de façon de telle sorte que, lorsqu'elle est activée, le faisceau lumineux incident traverse la membrane distale avant d'atteindre la membrane d'interface.

11. Dispositif selon l'une quelconque des revendications précédentes, dans lequel :
- la cavité est délimitée par une paroi distale (24) et une paroi latérale (22), la paroi latérale s'étendant entre la paroi distale (24) et la membrane d'interface ;
- la membrane d'interface s'étend entre des bords opposés de la paroi latérale.

12. Dispositif selon l'une quelconque des revendications précédentes, dans lequel le volume de la cavité est inférieur à 50 µL.

13. Dispositif selon l'une quelconque des revendications précédentes, dans lequel le détecteur acoustique est relié à la cavité par un canal acoustique.

14. Dispositif selon l'une quelconque des revendications précédentes, dans lequel la membrane d'interface est formée d'un matériau dont la conduction thermique est supérieure à 0.5 W.m⁻¹.K⁻¹.

15. Procédé de détection d'un analyte dans un milieu, l'analyte absorbant la lumière dans une longueur d'onde d'absorption, le procédé comportant :
- application d'un dispositif (1) selon l'une quelconque des revendications précédentes contre le milieu, de façon que la membrane d'interface soit au contact du milieu ;
- activation de la source de lumière (10), la bande spectrale d'émission contenant la longueur d'onde d'absorption de l'analyte ;
- détection d'une onde de pression photoacoustique par le détecteur acoustique et estimation d'une quantité d'analyte en fonction de l'onde de pression photoacoustique détectée.

16. Procédé selon la revendication 15, dans lequel le milieu (2) est liquide ou est un gel.

17. Procédé selon la revendication 15 ou la revendication 16, dans lequel la source de lumière est impulsionnelle ou modulée en amplitude, selon une fréquence d'impulsion ou une fréquence de modulation inférieure à 500 Hz.

## Patentansprüche

1. Photoakustische Detektionsvorrichtung (1), die dazu bestimmt ist, mit einer Kontaktseite (3) an ein zu analysierendes Medium (2) angelegt zu werden, wobei die Vorrichtung umfasst:
- eine hohle Kammer (20), die mit Gas gefüllt ist und an der Kontaktseite offen ist;
- eine Lichtquelle (10), die dazu ausgestaltet ist, wenn sie aktiviert ist, einen einfallenden Lichtstrahl (11) in einem Emissionsspektralband (Δλ) durch die Kammer (20) hindurch bis zu der Kontaktseite zu emittieren, wobei der einfallende Lichtstrahl impulsartig oder amplitudenmoduliert ist;
- einen akustischen Detektor (28), der mit der Kammer verbunden ist;
so dass, unter der Wirkung einer Beleuchtung des Mediums durch den einfallenden Lichtstrahl, der akustische Detektor eine akustische Welle detektiert, die durch eine Erwärmung des Mediums (2) erzeugt wird;
wobei die Vorrichtung eine Grenzflächenmembran (23) umfasst, welche die Kontaktseite bildet, wobei die Grenzflächenmembran dazu ausgestaltet ist:
- eine Grenzfläche zwischen dem die Kammer ausfüllenden Gas und dem zu analysierenden Medium zu bilden;
- einen Übergang des zu analysierenden Mediums in die Kammer zu blockieren;
**dadurch gekennzeichnet, dass** der akustische Detektor dazu ausgestaltet ist, eine akustische Druckwelle zu detektieren, die im Inneren der Kammer unter der Wirkung einer Änderung der Temperatur der Grenzflächenmembran erzeugt wird, wobei die Änderung der Temperatur der Grenzflächenmembran durch die aus der Beleuchtung des Mediums resultierende Erwärmung des Mediums verursacht wird.

2. Vorrichtung nach Anspruch 1, wobei die Grenzflächenmembran (23) durchgehend ist.

3. Vorrichtung nach Anspruch 1, wobei die Grenzflächenmembran durchgehende Öffnungen (23ₒ) mit einem Radius von weniger als 50 µm oder als 30 µm umfasst.

4. Vorrichtung nach Anspruch 3, wobei die Grenzflächenmembran an den durchgehenden Öffnungen eine hydrophobe Beschichtung umfasst.

5. Vorrichtung nach einem der vorhergehenden Ansprüche, wobei
- die Lichtquelle so angeordnet ist, dass, wenn sie aktiviert ist, der einfallende Lichtstrahl die Grenzflächenmembran durchquert, bevor er das zu analysierende Medium erreicht;
- die Grenzflächenmembran einen Kreuzungsabschnitt (23ᵢₙₜ) umfasst, der einem Teil der Membran entspricht, der von dem Lichtstrahl durchquert wird;
- die Grenzflächenmembran mindestens in dem Kreuzungsabschnitt aus einem Transmissionsmaterial besteht, das einen Transmissionsgrad in dem Emissionsspektralband von mehr als 0,4 aufweist.

6. Vorrichtung nach Anspruche 5, wobei das Transmissionsmaterial aus mindestens einem Material besteht, das gewählt ist aus: Si, Ge, AIN, ZnSe, BaF₂, CaF₂, KBr, ZnS, Saphir.

7. Vorrichtung nach einem der vorhergehenden Ansprüche, wobei die Grenzflächenmembran entnehmbar ist.

8. Vorrichtung nach einem der vorhergehenden Ansprüche, wobei
- die Grenzflächenmembran sich zwischen einer inneren Fläche (23ᵢ), die in Kontakt mit dem die Kammer ausfüllenden Gas ist, und einer äußeren Fläche (23ₑ), die dazu bestimmt ist, an das zu analysierende Medium angelegt zu werden, erstreckt;
- die innere Fläche der Grenzflächenmembran eine Antireflexbeschichtung umfasst oder eine Mikrostrukturierung, die dazu ausgestaltet ist, eine Reflexion des Lichtstrahls zu minimieren;
- und/oder die äußere Fläche der Grenzflächenmembran eine Antireflexbeschichtung umfasst oder eine Mikrostrukturierung, die dazu ausgestaltet ist, eine Reflexion des Lichtstrahls zu minimieren.

9. Vorrichtung nach einem der vorhergehenden Ansprüche, wobei die Dicke der Grenzflächenmembran zwischen 20 µm und 1 mm beträgt.

10. Vorrichtung nach einem der vorhergehenden Ansprüche, wobei:
- die Kammer (20) durch eine distale Membran begrenzt wird, wobei sich die distale Membran (24) gegenüber der Grenzflächenmembran erstreckt, so dass sich die Kammer zwischen der distalen Membran und der Grenzflächenmembran erstreckt;
- die Lichtquelle (10) so angeordnet ist, dass, wenn sie aktiviert ist, der einfallende Lichtstrahl die distale Membran durchquert, bevor er die Grenzflächenmembran erreicht.

11. Vorrichtung nach einem der vorhergehenden Ansprüche, wobei:
- die Kammer durch eine distale Wand (24) und eine Seitenwand (22) begrenzt wird, wobei sich die Seitenwand zwischen der distalen Wand (24) und der Grenzflächenmembran erstreckt;
- die Grenzflächenmembran sich zwischen entgegengesetzten Rändern der Seitenwand erstreckt.

12. Vorrichtung nach einem der vorhergehenden Ansprüche, wobei das Volumen der Kammer weniger als 50 µL beträgt.

13. Vorrichtung nach einem der vorhergehenden Ansprüche, wobei der akustische Detektor mit der Kammer durch einen akustischen Kanal verbunden ist.

14. Vorrichtung nach einem der vorhergehenden Ansprüche, wobei die Grenzflächenmembran aus einem Material gebildet ist, dessen Wärmeleitung mehr als 0,5 W.m⁻¹.K⁻¹ beträgt.

15. Verfahren zur Detektion eines Analyten in einem Medium, wobei der Analyt das Licht in einer Absorptionswellenlänge absorbiert, das Verfahren umfassend:
- Anlegen einer Vorrichtung (1) nach einem der vorhergehenden Ansprüche an ein Medium, so dass die Grenzflächenmembran in Kontakt mit dem Medium ist;
- Aktivieren der Lichtquelle (10), wobei das Emissionsspektralband die Absorptionswellenlänge des Analyten enthält;
- Detektion einer photoakustischen Druckwelle durch den akustischen Detektor und Schätzen einer Analytmenge in Abhängigkeit von der detektierten photoakustischen Druckwelle.

16. Verfahren nach Anspruch 15, wobei das Medium (2) flüssig ist oder ein Gel ist.

17. Verfahren nach Anspruch 15 oder Anspruch 16, wobei die Lichtquelle impulsartig oder amplitudenmoduliert ist, gemäß einer Impulsfrequenz oder einer Modulationsfrequenz von weniger als 500 Hz.

## Claims

1. A photoacoustic detecting device (1), intended to be applied, via a contact face (3), against a medium to be analyzed (2), the device comprising:
- a hollow cavity (20) that is filled with a gas, and that opens onto the contact face;
- a light source (10) that is configured to emit, when it is activated, an incident light beam (11), in an emission spectral band (Δλ), through the cavity (20), to the contact face, the incident light beam being pulsed or amplitude-modulated;
- an acoustic detector (28) connected to the cavity;
such that, under the effect of illumination of the medium by the incident light beam, the acoustic detector detects an acoustic wave produced by heating of the medium (2);
the device comprising an interface membrane (23), forming the contact face, the interface membrane being configured to:
- form an interface between the gas, filling the cavity, and the medium to be analyzed;
- block passage of the medium to be analyzed into the cavity;
**characterized in that**
the acoustic detector is configured to detect an acoustic pressure wave, generated inside the cavity, under the effect of a variation in the temperature of the interface membrane, the temperature variation of the interface membrane being induced by the heating of the medium resulting from the illumination of the medium.

2. The device as claimed in claim 1, wherein the interface membrane (23) is unapertured.

3. The device as claimed in claim 1, wherein the interface membrane comprises through-apertures (23ₒ) of radius less than 50 µm or than 30 µm.

4. The device as claimed in claim 3, wherein the interface membrane comprises a hydrophobic coating in the through-apertures.

5. The device as claimed in any one of the preceding claims, wherein:
- the light source is arranged such that, when it is activated, the incident light beam passes through the interface membrane before reaching the medium to be analyzed;
- the interface membrane comprises an intersecting segment (23ᵢₙₜ), corresponding to a portion of the membrane passed through by the light beam;
- at least in the intersecting segment, the interface membrane is made of a transmissive material having a transmittance higher than 0.4 in the emission spectral band.

6. The device as claimed in claim 5, wherein the transmissive material is at least one material selected from: Si, Ge, AIN, ZnSe, BaF₂, CaF₂, KBr, ZnS, sapphire.

7. The device as claimed in any one of the preceding claims, wherein the interface membrane is removable.

8. The device as claimed in any one of the preceding claims, wherein:
- the interface membrane extends between an internal surface (23ᵢ), making contact with the gas filling the cavity, and an external surface (23ₑ), intended to be applied against the medium to be analyzed;
- the internal surface of the interface membrane comprises an anti-reflection coating or micro-structuring configured to minimize reflection of the light beam;
- and/or the external surface of the interface membrane comprises an anti-reflection coating or micro-structuring configured to minimize reflection of the light beam.

9. The device as claimed in any one of the preceding claims, wherein the thickness of the interface membrane is comprised between 20 µm and 1 mm.

10. The device as claimed in any one of the preceding claims, wherein:
- the cavity (20) is bounded by a distal membrane, the distal membrane (24) lying opposite the interface membrane, so that the cavity extends between the distal membrane and the interface membrane;
- the light source (10) is arranged in such a way that, when it is activated, the incident light beam passes through the distal membrane before reaching the interface membrane.

11. The device as claimed in any one of the preceding claims, wherein:
- the cavity is bounded by a distal wall (24) and a lateral wall (22), the lateral wall extending between the distal wall (24) and the interface membrane;
- the interface membrane extends between opposite edges of the sidewall.

12. The device as claimed in any one of the preceding claims, wherein the volume of the cavity is less than 50 µL.

13. The device as claimed in any one of the preceding claims, wherein the acoustic detector is connected to the cavity by an acoustic channel.

14. The device as claimed in any one of the preceding claims, wherein the interface membrane is formed from a material the thermal conductivity of which is higher than 0.5 W.m⁻¹.K⁻¹.

15. A method for detecting an analyte in a medium, the analyte absorbing light at an absorption wavelength, the method comprising:
- applying a device (1) as claimed in any one of the preceding claims against the medium, so that the interface membrane makes contact with the medium;
- activating the light source (10), the emission spectral band containing the absorption wavelength of the analyte;
- detecting a photoacoustic pressure wave by means of the acoustic detector and estimating an amount of analyte depending on the detected photoacoustic pressure wave.

16. The method as claimed in claim 15, wherein the medium (2) is liquid or is a gel.

17. The method as claimed in claim 15 or claim 16, wherein the light source is pulsed or amplitude-modulated, with a pulse frequency or modulation frequency less than 500 Hz.
